# EUROPEAN PATENT SPECIFICATION

(11) **EP 2 786 719 B1**
(45) Date of publication and mention of the grant of the patent: **15.03.2017**
(21) Application number: 14174894.7
(22) Date of filing: 21.05.2007
(51) Int. Cl.: A61B 17/80, A61B 17/70

(54) **Bone fixation device**
Vorrichtung zur Knochenfixierung
Dispositif de fixation d'os

(30) Priority: 26.05.2006 GB 0610630
(43) Date of publication of application: 08.10.2014
(62) Divisional of application: 07732933.2
(73) Proprietor: Cunliffe, Mark, Richard, Edgerton Huddersfield West Yorkshire HD1 5QB (GB); Ness, Malcolm, Graham, Longframlington Morpeth Northumberland NE65 9EB (GB)
(72) Inventor: Cunliffe, Mark, Richard, Edgerton Huddersfield West Yorkshire HD1 5QB (GB); Ness, Malcolm, Graham, Longframlington Morpeth Northumberland NE65 9EB (GB)
(74) Representative: McDonough, Jonathan

(56) References cited:
- EP-A- 0 491 983
- DE-U1- 9 004 960
- GB-A- 2 405 342
- US-A- 6 022 350
- US-A1- 2003 074 004
- US-A1- 2003 153 912
- US-A1- 2004 111 089
- US-A1- 2005 015 090
- US-A1- 2005 065 515
- US-A1- 2006 009 771
- US-B1- 6 730 091

## Description

The present invention relates to a bone fixation device. More particularly, but not exclusively, the present invention relates to a bone fixation device comprising first and second screw receiving members connected together by a relatively pliable connecting arm adapted to bend before the screw receiving members.

Bone fixation plates are known. Such plates typically comprise a plastically deformable plate such as a metal plate. A plurality of apertures extend through the plate. In use the plate is positioned against a bone and screws passed through the apertures to fix the plate to the bone.

In order to ensure a close fit between the plate and the bone it is often necessary to deform the plate slightly. Deforming the plate distorts the apertures, this reduces the accuracy of the fit between plate and screws and may allow the plate to move relative to the bone during use.

A further bone fixation device is disclosed in PCT/US2005/020157. Such a device comprises a number substantially spherical screw receiving members having screws extending therethrough. Extending from each screw receiving member is a connecting member. A connecting arm, rather than extending along a long axis between the screw receiving members, extends to the side of the long axis. The screw receiving members are connected to the connecting arm by the connection members. Such a construction has a number of drawbacks. It is complex having a number of parts which must be assembled in a multistep procedure. In addition, one cannot simply twist the device about the long axis as is often required.

GB2405342A discloses a fibre reinforced bone fixation plate having a fixation zone for receiving a bone fixing such as a bone screw or nail. The bone plate may comprise a main body in which are fixed a plurality of inserts. Alternatively it may comprise a laminated structure with one or more layers of fibre reinforced material, the fixation zone being formed in the unreinforced material.

The device according to the invention seeks to overcome these problems.

### Accordingly, the present invention provides

A bone fixation device comprising
first and second screw receiving members, each member having an aperture extending therethrough for receiving a screw; and,
a plastically deformable connecting arm extending between the screw receiving members along a long axis defined by the first and second screw receiving members;
the arm being more pliable than the screw receiving members such that (a) on twisting the device about its long axis the arm twists before the screw receiving members deform; and (b) on bending the device along its long axis the arm bends before the screw receiving members deform;
at least one aperture being cylindrical and a part of the aperture being threaded for receiving a screw; the cylindrical aperture having at least one step in cross section along its length to define a lip for engagement with a tapered head of a screw.

The device according to the invention can be attached by a simplified procedure whereby it is deformed to the correct shape without deforming the apertures, placed against the bone and then screwed in place. In addition, it can be both bent along its long axis and twisted about its long axis to correctly align the device with the bone to the patient.

The device according to the invention is deformed to fit a bone the connecting arm(s) will deform before the screw receiving members. This allows the device to be deformed to the correct shape without the apertures becoming distorted.

Preferably, the device comprises a plurality of screw receiving members connected together in a line by connecting arms.

Preferably, at least one connecting arm is cylindrical in cross section.

At least one screw receiving member can be substantially spherical.

Preferably, a portion of the spherical member is flattened.

The flattened part can be centered about a mouth of the aperture and is in a plane normal to the axis of the aperture.

The present invention will now be described by way of example only and not in any limitative sense with reference to the accompanying drawings in which
Figure 1 shows a known fixation plate before and after deformation;
Figure 2 shows a further known bone fixation device in perspective view;
Figure 3 shows a bone fixation device not according to the invention in perspective view;
Figure 4 shows an embodiment of a bone fixation device according to the invention in combination with a screw;
Figure 5 shows a further embodiment of a bone fixation device according to the invention in cross sectional view; and
Figure 6 shows the embodiment of Figure 5 after twisting.

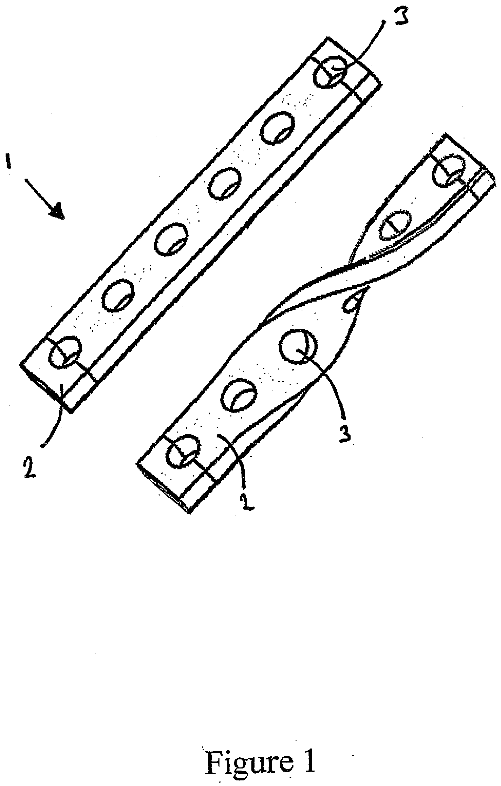

Shown in Figure 1 is a known bone plate 1. The bone plate 1 comprises a metal plate 2 having a plurality of apertures 3. In use the bone plate 1 is placed against a bone (not shown). Bone fixation screws (not shown) are passed through the apertures 3 and then screwed into the bone. The screw heads typically engage with the plate 1 firmly fixing the plate 1 in position and preventing it from being displaced with respect to the bone.

A problem can arise however if the bone plate 1 needs to be deformed before it can be fixed to the bone. Deformation of the plate 1 deforms the apertures 3 in the plate 1 as shown. This prevents the screw heads from accurately engaging with the apertures 3 which may result in the plate 1 being free to wobble slightly with respect to the bone. This can reduce the effectiveness of the bone plate 1 as a support for the bone.

In addition, it can be difficult to remove such a known bone plate 1 from the bone when it is no longer required. The screw heads tend to cold weld to the bone plate 1 over time making the screws difficult to remove. It is often necessary to cut the bone plate 1 free which can result in damage to the bone.

Such a known bone plate 1 is also limited as to how it can be deformed. Whilst the plate 1 can be bent as shown in Figure 1 it is not a simple matter to twist it such that the apertures 3 lie in different planes.

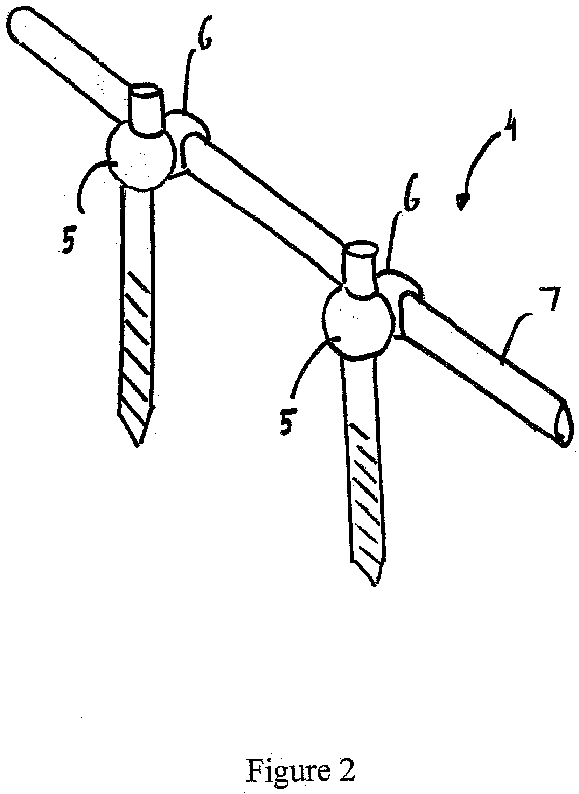

Shown in Figure 2 is a further known bone fixation device 4. This device 4 comprises a plurality of substantially spherical screw receiving members 5. Extending from each screw receiving member 5 is a connecting member 6. A connecting arm 7 extends through the connecting members 6 connecting the screw receiving members 5 together.

In use the screw receiving members 5 are arranged in the correct position and screwed to the bone. The connecting arm 7 is then passed through the connecting members 6 to complete the device 4.

Such a device 4 overcomes the problem of deformation of the screw receiving members by separating the screw receiving members 5 from the connecting arm 7 to be deformed. This separation however increases the complexity of the device 4 and the installation procedure. The connecting arm 7 needs to be deformed to a complex shape before it can be threaded through the connecting members 6. The threading can be difficult or even impossible in a confined space. Because of the gap between the screw receiving members 5 and the connecting arm 7 the device 4 is unsuitable for use where a longitudinal twist of the device 4 is required.

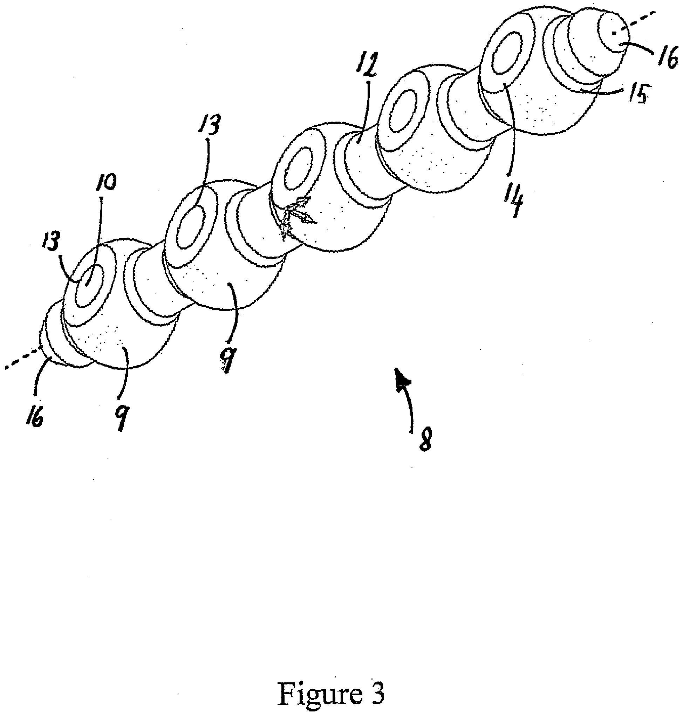

Shown in Figure 3 is a bone fixation device 8 which is not according to the invention. The device comprises a plurality of screw receiving members 9. Apertures 10 extend through each of the screw receiving members 9 for receiving screws. A long axis 11 extends between each of the screw receiving members 9. Plastically deformable connecting arms 12 extend along the long axis 11 between the screw receiving members 9 as shown. In this embodiment the long axis 11 passes through the apertures 10 of the screw receiving members 9.

In use the bone fixation device 8 is gripped and bent to the required shape. The arms 12 are more pliable than the screw receiving members 9 and accordingly it is the arms 12 that bend when the force is applied, rather than the screw receiving members 9.

The apertures 10 therefore remain undistorted. In addition, in contrast to known bone plates 1 a torsional (twisting) force can be applied to the device 8 rotating one or more of the screw receiving members 9 about the long axis 11 of the device 8 if required. As the long axis 11 passes along the length of the connecting arm 12 the connecting arm 12 twists about its length. The device 8 can therefore be twisted without significantly altering its dimensions. The device 8 can therefore be inserted into small apertures even after twisting.

Each of the screw receiving members 9 is substantially spherical with the apertures 10 extending through the centres of the spheres 9. Each aperture 10 intersect the sphere at mouths 13 on opposite sides of the sphere 9. The sphere 9 comprises a slightly flattened portion 14 around one of the mouths. This reduces the profile of the device 8. It also provides an extended contact area between the screw receiving members 9 and the bone (not shown).

The connecting arms 12 between the screw receiving members 9 are cylindrical. The interface 15 between the arms 12 and spherical screw receiving members 9 is chamfered so that any bending or torsional forces do not concentrate at this interface 15.

The ends 16 of the device 8 are tapered as shown so that the device 8 can be placed between bone and soft tissue without surgically exposing the entire length of bone.

Each of the apertures 10 of this embodiment is cylindrical having a constant area along its length. A portion of the aperture 10 is threaded. The remainder of the aperture 10 is smooth walled.

After bending and/or twisting to the correct shape the device 8 is positioned against the bone. Screws (not shown) are inserted into the apertures 10 through the smooth portions and into threaded engagement with the threaded portions. On further rotation of the screws they penetrate and grip the bone, fixing the device 8 to the bone. A significant advantage of the device 8 is that it can be bent/twisted to the correct shape, positioned correctly and then screws inserted. This considerably simplifies the attachment procedure. As the screw receiving members 9 are aligned with the connection arms 12 along the long axis 11 the device 8 can be twisted about its length without any significant change in dimensions of the device 8. This is particularly useful when inserting the device 8 into small apertures.

The device 8 is adapted to be used with a screw (not shown) having two portions - a threaded portion for gripping the threaded portion of the aperture 10 and then the bone and a smooth portion extending from the threaded portion. The smooth portion has an outer face which is substantially cylindrical and of the same diameter as the threaded portion. The smooth portion is however slightly tapered with its diameter increasing in a direction away from the threaded portion. At its end the diameter of the smooth portion is slightly larger than the diameter of the aperture 10. As the screw is turned and is drawn into the aperture 10 the smooth portion of the screw abuts the smooth portion of the aperture 10 so producing a press fit.

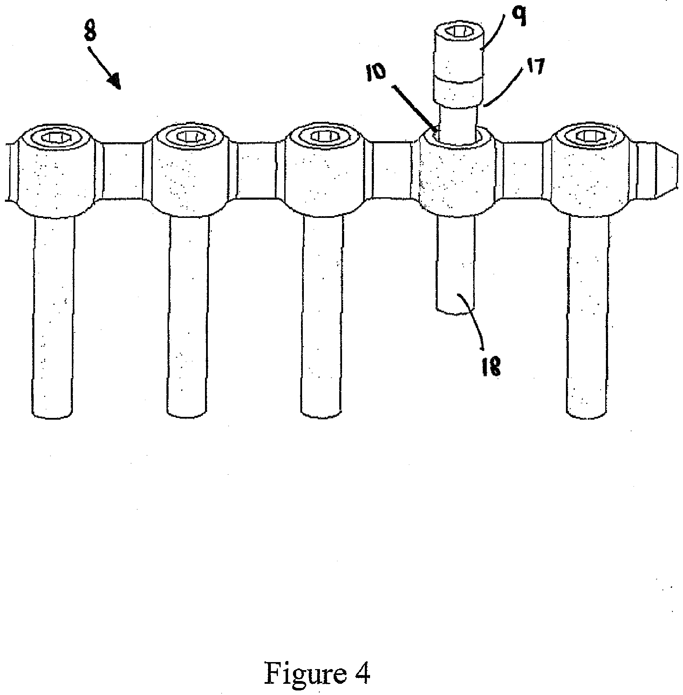

An embodiment of a device according to the invention is shown in Figure 4. In contrast to the device of Figure 3 the threaded portion of the aperture 10 is narrower in diameter than the smooth portion.

The embodiment of Figure 4 can be used with an alternative design of screw (not shown). The alternative design comprises a threaded shaft having a domed head extending away from the shaft. As the screw is drawn into the aperture 10 the domed head abuts the lip formed by the change in diameter of the aperture 10.

In a further embodiment of the invention (not shown) both the narrow and wide portions of the aperture 10 are threaded. Similarly, the screw comprises a narrow threaded shaft and a larger diameter threaded head. In use the threaded head engages with the larger portion of the aperture 10.

In a further embodiment of the invention (not shown) a portion of the aperture 10 is slightly bevelled so that a standard bone screw head will press fit into engagement with the bevels as the screw is drawn into the aperture 10.

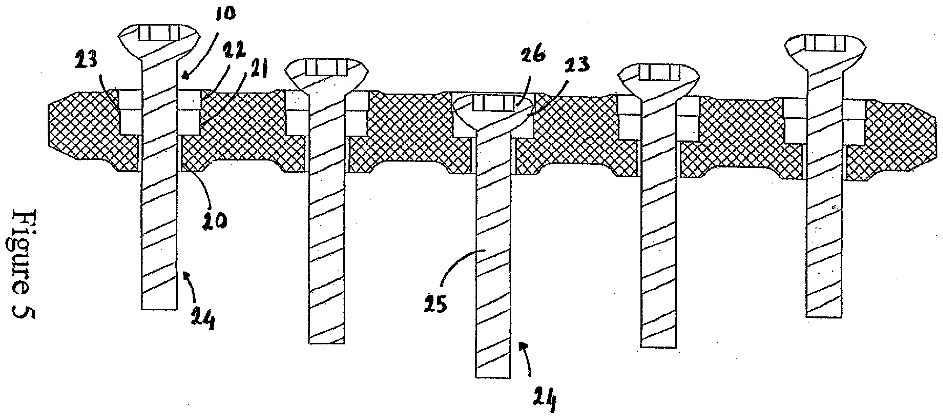

Shown in Figure 5 in cross sectional view is a further embodiment of a device 8 according to the invention. The aperture 10 comprises a first narrow threaded section 20. Extending from this is a second smooth walled section 21 of larger diameter. Extending from the second section 21 is a third smooth walled section 22 of slightly larger diameter then the second section 21. The step change in diameter from the second section 21 to the third section 22 defines a lip 23. In use a screw 24 is inserted into the aperture 10 with the threaded portion 25 of the screw 24 in threaded engagement with the narrower portion 21 of the aperture 10. As the screw 24 is turned and drawn into the aperture 10 the head 26 of the screw 24 abuts the lip 23 producing a tight fit.

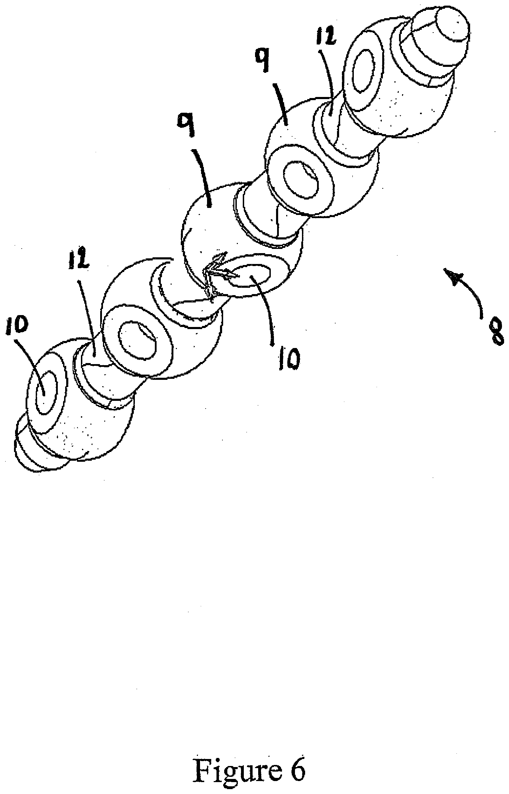

Shown in Figure 6 is the embodiment of Figure 5 in perspective view. The device 8 has been twisted along its length such that the apertures 10 lie in different planes. As can be seen, the arms 12 have twisted before the screw receiving members 9 deform.

In a further embodiment of the invention the screw receiving members 9 are substantially elliptical.

The device 8 according to the invention can be used with any tool which grips the screw receiving members 9. One preferred embodiment of such a tool comprises jaws having cut out sections which match the spherical component of the screw receiving members 9. The tool also has cut outs which match the interface 15 between the spherical component 9 and connecting arm 12 so that the tool can apply a bending force throughout the length of the bone fixation device 8. In an alternative embodiment the jaws have cut outs which match the flattened portions 14 of the screw receiving members 9.

In use two of the tools are used to grip the screw receiving members 9. The device 8 is then bent and/or twisted to the desired shape and then released.

## Claims

1. A bone fixation device (8) comprising
first and second screw receiving members (9), each member (9) having an aperture (10) extending therethrough for receiving a screw; and,
a plastically deformable connecting arm (12) extending between the screw receiving members (9) along a long axis defined by the first and second screw receiving members (9);
the arm (12) being more pliable than the screw receiving members (9) such that (a) on twisting the device (8) about its long axis the arm (12) twists before the screw receiving members (9) deform; and (b) on bending the device (8) along its long axis the arm (12) bends before the screw receiving members (9) deform;
at least one aperture (10) being cylindrical and a part of the aperture (10) being threaded for receiving a screw;
the cylindrical aperture (10) having at least one step in cross section along its length to define a lip for engagement with a tapered head of a screw.

2. A bone fixation device (8) as claimed in claim 1, comprising a plurality of screw receiving members (9) connected together in a line by connecting arms (12)

3. A bone fixation device (8) as claimed in either of claims 1 or 2, wherein at least one connecting arm (12) is cylindrical in cross section

4. A bone fixation device (8) as claimed in any one of claims 1 to 3, wherein at least one screw receiving member (9) is substantially spherical

5. A bone fixation device (8) as claimed in claim 4, wherein a portion (14) of the spherical screw receiving member (9) is flattened.

6. A bone fixation device (8) as claimed in claim 5, wherein the flattened portion (14) is centered about a mouth (13) of the aperture (10) and is in a plane normal to the axis of the aperture (10).

## Patentansprüche

1. Knochenfixiervorrichtung (8) umfassend;
ein erstes und ein zweites Schraubenaufnahmeelement (9), wobei jedes Element (9) eine Öffnung (10) umfasst, die sich durch dieses erstreckt, um eine Schraube aufzunehmen; und
einen plastisch verformbaren Verbindungsarm (12), der sich zwischen den Schraubenaufnahmeelementen (9) entlang einer Längsachse erstreckt, welche von dem ersten und zweiten Schraubenaufnahmeelement (9) definiert wird;
wobei der Arm (12) derart verformbarer als die Schraubenaufnahmeelemente (9) ist, dass (a) beim Verdrehen der Vorrichtung (8) um deren Längsachse der Arm (12) sich verdreht, bevor sich die Schraubenaufnahmeelemente (9) verformen und dass (b) beim Biegen der Vorrichtung (8) der Arm (12) sich entlang dessen Längsachse verbiegt, bevor sich die Schraubenaufnahmeelemente (9) verformen;
wenigstens eine Öffnung (10) zylindrisch ist und ein Teil der Öffnung (10) mit einem Gewinde versehen ist, um eine Schraube aufzunehmen;
wobei die zylindrische Öffnung (10) wenigstens eine Stufe im Querschnitt entlang deren Länge aufweist, um eine Lippe für die Ineingriffnahme mit einem verjüngten Kopf einer Schraube zu definieren.

2. Knochenfixiervorrichtung (8) nach Anspruch 1, umfassend eine Vielzahl vom Schraubenaufnahmeelementen (9), die in einer Linie durch die Verbindungsarme (12) miteinander verbunden sind.

3. Knochenfixiervorrichtung (8) nach Anspruch 1 oder 2, wobei wenigstens ein Verbindungsarm (12) einen zylindrischen Querschnitt aufweist.

4. Knochenfixiervorrichtung (8) nach einem der Ansprüche 1 bis 3, wobei wenigstens ein Schraubenaufnahmeelement (9) im Wesentlichen kugelförmig ist.

5. Knochenfixiervorrichtung (8) nach Anspruch 4, wobei ein Abschnitt (14) des kugelförmigen Schraubenaufnahmeelements (9) abgeflacht ist.

6. Knochenfixiervorrichtung (8) nach Anspruch 5, wobei der abgeflachte Abschnitt (14) um eine Mündung (13) der Öffnung (10) zentriert ist und sich in einer Ebene senkrecht zu der Achse der Öffnung (10) befindet.

## Revendications

1. Dispositif (8) pour la fixation d'os comprenant :
un premier membre et un deuxième membre (9) récepteurs de vis, chaque membre (9) présentant une ouverture (10) qui s'étend à travers ledit membre pour la réception d'une vis ; et
un bras connecteur (12) à déformation plastique s'étendant entre les membres (9) récepteurs de vis le long d'un axe défini entre les premier et deuxième membres (9) récepteurs de vis ;
le bras (12) étant plus déformable que les membres (9) récepteurs de vis de telle sorte que :
(a) lors d'une torsion du dispositif (8) autour de son axe longitudinal, le bras (12) se tord avant que les membres (9) récepteurs de vis ne se déforment ; et
(b) lorsqu'on plie le dispositif (8) le long de son axe longitudinal, le bras (12) se plie avant que les membres (9) récepteurs de vis ne se déforment ;
au moins une ouverture (10) étant de forme cylindrique et une partie de l'ouverture (10) est filetée pour la réception d'une vis ;
l'ouverture cylindrique (10) présentant au moins un pas en section diamétrale le long de sa longueur afin de définir une lèvre d'engagement avec une tête conique d'une vis.

2. Dispositif (8) pour la fixation d'os selon la revendication 1, comprenant une pluralité de membres (9) récepteurs de vis connectés ensemble en ligne par des bras connecteurs (12).

3. Dispositif (8) pour la fixation d'os selon la revendication 1 ou la revendication 2, selon lequel au moins un bras connecteur (12) présente une section diamétrale de forme cylindrique.

4. Dispositif (8) pour la fixation d'os selon l'une quelconque des revendications 1 à 3, selon lequel au moins un membre (9) récepteur de vis est de forme sensiblement sphérique.

5. Dispositif (8) pour la fixation d'os selon la revendication 4, selon lequel une partie (14) du membre (9) sphérique récepteur de vis est aplatie.

6. Dispositif (8) pour la fixation d'os selon la revendication 5, selon lequel la partie aplatie (14) est centrée autour d'une embouchure (13) de l'ouverture (10) et se présente selon un plan normal relatif à l'axe de l'ouverture (10).
